# EUROPEAN PATENT SPECIFICATION

(11) **EP 1 630 136 B1**
(45) Date of publication and mention of the grant of the patent: **11.07.2012**
(21) Application number: 04724741.6
(22) Date of filing: 31.03.2004
(51) Int. Cl.: C09C 1/36, C09C 1/00, C01G 23/053, C01G 49/06, A61K 33/00, A61K 41/00, A61K 47/48, A61K 47/02, B01J 35/02

(54) **SURFACE-MODIFIED TITANIUM DIOXIDE FINE PARTICLES AND DISPERSION COMPRISING THE SAME, AND METHOD FOR PRODUCING THE SAME**
FEINE TEILCHEN AUS OBERFLÄCHENMODIFIZIERTEM TITANDIOXID UND DIESE ENTHALTENDE DISPERSION UND HERSTELLUNGSVERFAHREN DAFÜR
FINES PARTICULES DE DIOXYDE DE TITANE MODIFIEES EN SURFACE, DISPERSION COMPRENANT CELLES-CI, ET PROCEDE DE PRODUCTION DESDITES PARTICULES

(30) Priority: 31.03.2003 JP 2003094430; 30.09.2003 JP 2003340228
(43) Date of publication of application: 01.03.2006
(73) Proprietor: Toto Ltd., Kitakyushu City, Fukuoka 802-8601 (JP)
(72) Inventor: SONEZAKI, Shuji, c/o Toto Ltd., Kitakyushu-shi, Fukuoka 802-8601 (JP); BANZAI, Toshiaki, c/o Toto Ltd., Kitakyushu-shi, Fukuoka 802-8601 (JP); KANEHIRA, Koki, c/o Toto Ltd., Kitakyushu-shi, Fukuoka 802-8601 (JP); YAGI, Shinichi, c/o Toto Ltd., Kitakyushu-shi, Fukuoka 802-8601 (JP); OGAMI, Yumi, c/o Toto Ltd., Kitakyushu-shi, Fukuoka 802-8601 (JP)
(74) Representative: Gillard, Richard Edward
(86) International application number: PCT/JP2004/004635
(87) International publication number: WO 2004/087577

(56) References cited:
- EP-A- 1 273 636
- JP-A- 10 067 516
- JP-A- 2002 316 946
- JP-A- 2002 316 950
- PERRIN F X ET AL: "Preparation and properties of acrylic polymers/titania hybrid materials prepared by the in situ sol-gel process" POLYMER INTERNATIONAL, SOCIETY OF CHEMICAL INDUSTRY, vol. 51, no. 10, 1 October 2002 (2002-10-01), pages 1013-1022, XP002496732 ISSN: 0959-8103
- KHALED, S.M.; SUI, RUOHONG; CHARPENTIER, PAUL A.; RIZKALLA, AMIN S.: "Synthesis of TiO2-PMMA Nanocomposite: using methacrylic acid as a coupling agent" LANGMUIR, vol. 23, no. 7, 2007, pages 3988-3995, XP002507221

## Description

### TECHNICAL FIELD

The present invention relates to surface modified titanium dioxide fine particles comprising titanium dioxide having a surface modified with a carboxyl-containing hydrophilic polymer, carboxyl groups in the hydrophilic polymer having been bonded to titanium dioxide through an ester linkage, a dispersion comprising the same, and a process for producing the same.

### BACKGROUND ART

Titanium dioxide has hitherto been said to have an isoelectric point around pH 6, and due to this, titanium dioxide particles are disadvantageously agglomerated in a near-neutral aqueous solvent and thus could not have been homogeneously dispersed without significant difficulties. To overcome this problem, up to now, various studies have been made in order to homogeneously disperse titanium dioxide particles in an aqueous dispersant. For example, a proposal has been made on a nitric acid-containing acidic titanium dioxide sol produced by producing precipitates of titanium hydroxide from titanium isopropoxide and peptizing the precipitates under nitric acid-containing acidic conditions at an elevated temperature (see, for example, christophe, Barbe et al: Journal of the American Ceramics Society, 80, 3157-3171 (1997), and Danijela, Vorkapic et al: Journal of the American Ceramics Society, 81, 2815-2820(1998)). Other proposals include a method which comprises adding aqueous ammonia dropwise to an aqueous titanium tetrachloride solution to precipitate titanium hydroxide, then adding aqueous hydrogen peroxide, and allowing a reaction to proceed at 100°C for 6 hr to give a peroxo group-modified titanium dioxide sol comprising titanium dioxide particles the surface of which has been modified with a peroxo group (see, for example, Japanese Patent Laid-Open No. 67516/1998), a method for preparing a dispersion liquid of composite-type titanium dioxide fine particles, which comprises coating the surface of titanium dioxide particles with porous silica and dispersing the surface-coated titanium dioxide particles under alkaline conditions for stabilization (see, for example, Japanese Patent Laid-Open No. 319577/1999), and a method for preparing an aqueous solution of titanium dioxide having enhanced dispersiblity, which comprises incorporating a polycarboxylic acid or its salt as a dispersant (see, for example, Japanese Patent Laid-Open No. 212315/1990).

Further, proposals have been made on particles comprising a magnetic material composed with titanium dioxide for facilitating separation and concentration in the case where photocatalyst particles are used in water treatment. Examples thereof include particles the surface of which has been coated with a titanium alkoxide dissolved in an organic solvent using iron powder as a carrier (see, for example, Japanese Patent Laid-Open No. 299810/1997), and a method for preparing magnetic material/titanium dioxide composite particles, which comprises depositing amorphous or crystalline titanium dioxide directly on an iron oxide-silica carrier by high-temperature treatment (see, for example, Watson, Beydoun et al: Journal of Photochemistry and Photobiology A: Chemistry, 148, 303-313 (2002)).

The nitric acid-containing acidic titanium dioxide sol, however, suffers from a problem that, for example, when the pH value of the sol is adjusted to neutral or alkaline side, agglomeration or precipitation occurs. The peroxo group-modified titanium dioxide sol also suffers from a drawback that, for example, although the pH value of the sol is neutral, .. the addition of an inorganic salt in the sol disadvantageously causes agglomeration or precipitation. Further, in the case of the dispersion liquid of titanium dioxide fine particles the surface of which has been coated with porous silica, bringing pH value of the dispersion liquid to neutral or acidic side disadvantageously causes agglomeration or precipitation. Furthermore, the aqueous titanium dioxide solution in which the dispersant has been added for enhancing the dispersiblity may suffer from a problem that the dispersant is decomposed by the activity of the photocatalyst, or otherwise the activity of the photocatalyst is deteriorated. In addition, when the salt coexists, titanium dioxide disadvantageously causes agglomeration or precipitation. The same phenomenon takes place in the case of composite particles comprising titanium dioxide present on a part of the surface of the magnetic material. That is, also in this case, a problem of agglomeration and precipitation takes place.

On the other hand, an attempt has been made to apply titanium dioxide having a high level of photoactivation degradation activity to a drug delivery system (DDS) (see, for example, Japanese Patent Laid-Open No. 316946/2002, Japanese Patent Laid-Open No. 316950/2002, and R. Cai et al: Cancer Research, 52, 2346-2348 (1992)). In this method, particles of a metal such as gold supported on titanium dioxide are injected and incorporated in target cancer cells, followed by application of light such as ultraviolet light to kill the cancer cells. Titanium dioxide is known to be a material that is very stable in the air or solution and, at the same time, is nontoxic and safe within the body of an animal (i.e., in light shielded state). Further, since the activation of titanium dioxide can be controlled by on-off control of light, the application of titanium dioxide to DDS, for example, for cancer treatment purposes is expected.

As described above, however, the isoelectric point of titanium dioxide is around pH 6, and, thus, titanium dioxide particles are disadvantageously agglomerated under near-neutral physiological conditions. For this reason, it is impossible to administrate a dispersion liquid of titanium dioxide as an injection directly into blood vessels or use the titanium dioxide particles per se as a carrier for DDS.

FX Perrin et al, Polym Int 51, 1013-1022 (2002) discloses a hybrid inorganic-organic material based on a methyl methacrylate-n-butyl methacrylate-methacrylic acid terpolymer prepared by an in situ sol-gel process in the presence of tetrabutyltitanate. The process results in a homogenous and semi-transparent film with amounts of titania ranging from 4.5 to 22%. The titanium oxide produced is in the form of an amorphous network.

### DISCLOSURE OF THE INVENTION

The present inventors have made extensive and intensive studies with a view to solving the above problems and, as a result, have found that surface modification by chemically bonding a hydrophilic polymer onto the surface of titanium dioxide fine particles in an anatase or rutile form can highly improve dispersibility in aqueous solvents over a broad pH range including near-neutral pH. This has led to the completion of the present invention.

Specifically, the surface-modified titanium dioxide fine particles according to the present invention have on the surface thereof a hydrophilic polymer through an ester linkage and, thus, have very good dispersibility in aqueous solvents even in a broad range of pH including near-neutral pH. Further, a dispersion liquid of surface-modified titanium dioxide fine particles utilizing this feature can utilize water- or salt-containing various pH buffer solutions as solvents and has very good dispersibility and stability. The production process of surface-modified titanium dioxide fine particles according to the present invention comprises the steps of mixing a dispersion liquid of titanium dioxide particles having a size of 2 to 200 nm and a water soluble polymer solution together, heating the mixture at 80 to 220°C to form an ester bond between the titanium dioxide particles and the water soluble polymer, then removing the water soluble polymer remaining unbonded, and purifying the surface-modified titanium dioxide fine particles.

The surface-modified titanium dioxide fine particles according to the present invention thus obtained can be dispersed in an aqueous solvent over a broad range of pH including neutral pH and further is very stable against pH fluctuation and the addition of salt. Further, since compositing with other functional substances is easy, the surface-modified titanium dioxide fine particles according to the present invention is effective in preparing novel function-imparted particles. For example, the surface-modified titanium dioxide fine particles according to the present invention can be utilized in the development of DDS in which an anti-cancer drug is supported on the surface modified titanium dioxide fine particles according to the present invention and the anti-cancer drug is released by a photoswitch. Further, introduction of the surface-modified titanium dioxide fine particles according to the present invention directly into an affected region in the body followed by application of light such as ultraviolet light can efficiently destroy cancer tissues and the like because agglomeration does not take place. Furthermore, application of ultraviolet light, sunlight or the like to induce photocatalytic activity-derived redox action can realize degradation of various organic matter and microorganisms.

### BRIEF DESCRIPTION OF THE DRAWINGS

Fig. 1 is a typical diagram showing the surface-modified titanium dioxide fine particles according to the present invention;
Fig. 2 is a diagram showing the results of measurement of photocatalytic activity of the surface-modified titanium dioxide fine particles according to the present invention (expressed in terms of a reduction in absorbance involved in the deposition of methylene blue), wherein ○, ●, □, ■, and Δ represent polyacrylic acid-bonded titanium dioxide fine particles (anatase form) prepared in Examples 1 to 5; and
Fig. 3 is a graph showing cytotoxic activity, derived from photocatalystic activity of the surface-modified titanium dioxide fine particles according to the present invention, against cancer cells.

### BEST MODE FOR CARRYING OUT THE INVENTION

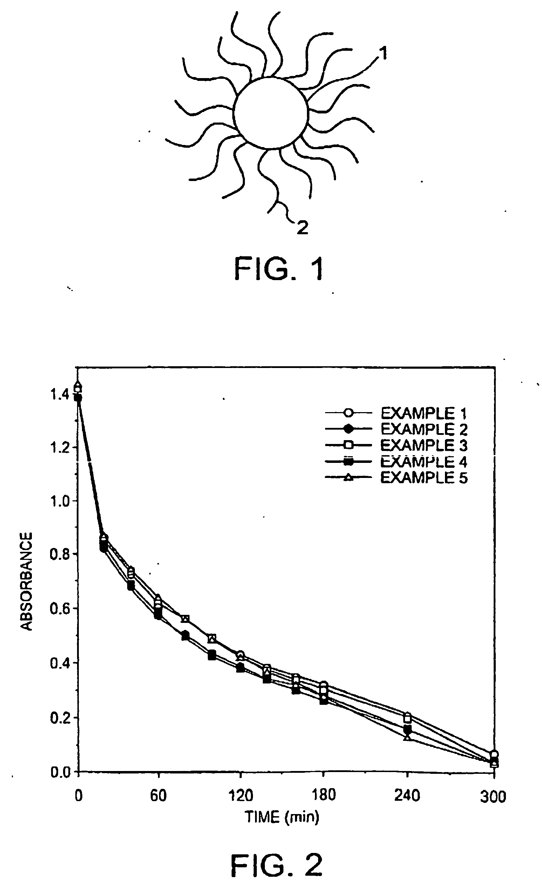

Embodiments of the present invention will be described in detail with reference to the accompanying drawings. Fig. 1 is a typical diagram showing surface-modified titanium dioxide fine particles according to the present invention. The surface-modified titanium dioxide fine particles 1 according to the present invention are characterized by comprising a hydrophilic polymer 2 present on the surface of titanium dioxide fine particles, carboxyl groups in the hydrophilic polymer 2 having been bonded to titanium dioxide through an ester linkage. This is realized by hydrating titanium dioxide on the surface of the titanium dioxide fine particles 1 with water in the reaction system to produce hydroxyl groups which are then reacted with carboxyl groups in the hydrophilic polymer to form an ester linkage. The formation of the ester linkage can be confirmed by various analytical methods. For example, the ester linkage can be confirmed by providing an infrared spectrum by infrared spectroscopy and observing the spectrum for infrared absorption around 1700 to 1800 cm⁻¹, an absorption band characteristic of an ester linkage. Upon this surface modification, the isoelectric point of the surface modified titanium dioxide fine particles are brought to a value around the isoelectric point of the carboxyl residue of the hydrophilic polymer (pH 2.8 to 2.9), and, thus, electrical repulsive force is applied among particles even in a neutral aqueous solvent, resulting in good dispersibility.

In the titanium dioxide particles used in the present invention, the crystal system is of any of anatase form and rutile form, because, even in the case of different crystal systems, the surface modification is possible so far as they are identical to each other in a chemical property that a hydroxyl group is produced as a result of hydration. When a high level of photocatalytic activity is desired, anatase form may be formed, while, when properties such as a high refractive index for applications such as cosmetic preparations are desired, rutile form may be selected. For the same reason as described above, not only single titanium dioxide particles but also composite titanium dioxide particles comprising titanium dioxide and a magnetic material can be advantageously used. The diameter of dispersed particles is preferably 2 to 200 nm from the viewpoint of degree of freedom of usage. When the particle diameter is larger than 200 nm, the effect of gravity acting on the fine particles is increased and, consequently, the particles are more likely to settle.

The surface-modified titanium dioxide fine particles according to the present invention are further characterized in that the hydrophilic polymer is a water soluble polymer. In the present invention, the surface-modified titanium dioxide fine particles are contemplated to be used in a dispersion in an aqueous solution, and, thus, a water soluble polymer is preferred as the hydrophilic polymer used in the present invention. Any water soluble polymer may be used so far as it contains a plurality of carboxyl groups. Examples of water soluble polymers usable herein include carboxymethyl starch, carboxymethyl dextran, carboxymethylcellulose, polycarboxylic acids, and copolymers containing carboxyl units. More specifically, polycarboxylic acids such as polyacrylic acid and polymaleic acid, and copolymers such as acrylic acid/maleic acid copolymer and acrylic acid/sulfonic acid monomer copolymer are more preferred from the viewpoint of hydrolyzability and solubility of water soluble polymer.

The dispersion liquid of the surface-modified titanium dioxide fine particles according to the present invention is characterized in that the surface modified titanium dioxide fine particles are dispersed in an aqueous solvent. In the aqueous dispersion medium, since protons of carboxyl residues present on surface modified titanium dioxide fine particles are in a dissociated state, electrical repulsive force is applied among particles and, thus, the dispersion liquid of surface modified titanium dioxide fine particles according to the present invention is stably present without causing agglomeration for a long period of time. Further, basically, the dispersion liquid of surface modified titanium dioxide fine particles according to the present invention is also very stable against pH fluctuation and the addition of inorganic salts. Furthermore, it should be noted that the isoelectric point of the surface modified titanium dioxide fine particles according to the present invention is around the isoelectric point of the carboxyl residue of the hydrophilic polymer (pH 2.8 to 2.9). Accordingly, in an aqueous dispersion medium having a pH value of 3 or more, the electrical repulsive force applied among the particles increases with increasing the pH value, and, thus, good dispersibility can be realized in an aqueous dispersion medium having a pH value of 3 to 13. For the above reasons, in this dispersion liquid, pH buffer solution can be utilized as the aqueous solvent. Specifically, the surface modified titanium dioxide fine particles according to the present invention have good dispersibility even when any buffer component is contained in the aqueous dispersion medium, so far as the pH value is in the range of 3 to 13. Suitable buffer solutions usable herein include glycine buffer solutions, acetate buffer solution, phosphate buffer solutions (including PBS), carbonate buffer solutions, Mcllvaine buffer solutions, Good's buffer solutions, and borate buffer solutions. That near-neutral buffer solutions are usable is very advantageous for applications in biotechnology and pharmaceutical and medical fields. In order to maintain good dispersibility, the ratio of amount of carboxyl group/titanium dioxide in the surface modified titanium dioxide fine particles in the dispersion liquid (mol/g) is preferably approximately not less than 2 × 10⁻³ although the ratio varies depending upon reaction conditions.

Further according to the present invention, there is provided a process for producing surface-modified titanium dioxide fine particles by chemically bonding a hydrophilic polymer to the surface of titanium dioxide fine particles, wherein said titanium dioxide is an anatase or rutile form of titanium dioxide said process being characterized by comprising:
(1) the first step of dispersing titanium dioxide sol in a solvent; (2) the second step of dispersing a hydrophilic polymer in a solvent; (3) the third step of mixing the two dispersion liquids together; (4) the fourth step of heating the mixed liquid; (5) the fifth step of separating the surface-modified titanium dioxide fine particles from the hydrophilic polymer remaining unbonded; and (6) the sixth step of purifying the surface-modified titanium dioxide fine particles.

The titanium dioxide sol used in the present invention may be synthesized using titanium tetraisopropoxide or the like as a starting compound, or alternatively may be an existing acidic titanium dioxide sol peptized with an inorganic acid. On the other hand, a solvent in which both the titanium dioxide sol and the hydrophilic polymer are soluble is suitable as the solvent used in steps (1) and (2). The reason for this is that, when titanium dioxide is agglomerated in the solvent, the surface area in which a binding reaction between titanium dioxide and the hydrophilic polymer can take place is reduced and, thus, the diameter of dispersed particles in the aqueous solvent after the completion of the reaction is increased, leading to deteriorated dispersibility. Further, it should be noted that a solvent reactive with the surface of the titanium dioxide particles is unsuitable as the solvent used herein. In particular, hydroxyl-containing alcohols, upon heating, combine with the surface of the titanium dioxide particles to from an ether linkage and, thus, inhibit the contemplated binding reaction between titanium dioxide and the hydrophilic polymer. In this case, surface properties of the titanium dioxide particles depend upon the properties of the alcohol used, and the dispersibility in aqueous dispersion media is significantly lowered. From the viewpoint of reactivity, aprotic polar solvents such as dimethylformamide, dioxane, or dimethylsulfoxide are preferably used as the solvent in the present invention. From the viewpoint of volatility of the solvent, the use of dimethylformamide is more preferred.

Next, in the step (3), the titanium dioxide dispersion liquid and hydrophilic polymer dispersion liquid using the above solvent are mixed together, and the mixture is stirred to prepare a dispersion liquid comprising homogeneously dispersed titanium dioxide and hydrophilic polymer. In this case, the addition of the hydrophilic polymer directly to the titanium dioxide dispersion liquid sometimes causes agglomeration of titanium dioxide. For this reason, preferably, the titanium dioxide dispersion liquid and the hydrophilic polymer dispersion liquid are separately prepared followed by mixing of these dispersions.

Next, in the step (4), the mixed liquid is heated for a binding reaction. In this case, the reaction proceeds without applying any pressure by properly selecting the ratio between the titanium dioxide and the hydrophilic polymer. Since, however, the application of pressure further promotes the reaction, preferably, the reaction is allowed to proceed under pressure. In this case, when polyacrylic acid (average molecular weight: 5000) is used as the hydrophilic polymer, the final concentration of polyacrylic acid is preferably brought to not less than 0.4 mg/ml from the viewpoint of realizing better dispersibility. The production process of the present invention is characterized in that the heating temperature is 80 to 220°C. When the heating temperature is below 80°C, the amount of the hydrophilic polymer bonded is lowered and, consequently, the dispersibility in the aqueous solvent is lowered. When the reaction is carried out under pressure, a heating temperature above 220°C is unsuitable from the viewpoint of the sealing property of the reaction vessel. Further, when a reaction is allowed to proceed at a temperature at or above the boiling point of water, upon full vaporization of water contained in the titanium dioxide sol to the outside of the reaction system, titanium dioxide is agglomerated and, thus, the reaction is preferably allowed to proceed under pressure. On the other hand, when the content of water in the reaction solution is excessively high, the reaction is sometimes inhibited by water. Therefore, the content of water in the reaction solution is preferably not more than about 4% although it varies depending upon reaction conditions.

Next, in step (5), the produced surface modified titanium dioxide fine particles are separated from the hydrophilic polymer remaining unbonded. For example, dialysis, ultrafiltration, gel permeation chromatography, or precipitation is suitable as the separation means. In the separation by dialysis or ultrafiltration, a dialytic membrane or an ultrafiltration membrane compatible with the molecular weight of the hydrophilic polymer used should be used. That is, separation can be carried out by any of the above methods. Preferably, the precipitation method is utilized from the viewpoint of simpleness of the operation. Any of a method utilizing an isoelectric point and a method utilizing salting out may be preferably used for the precipitation method.

Precipitation utilizing isoelectric point precipitation is carried out according to the following procedure. After the completion of the reaction, the reaction solvent is removed under reduced pressure by an evaporator, water is added to the residue, and the mixture is stirred to disperse surface modified titanium dioxide fine particles. When the dispersion liquid is adjusted to pH not more than 2.8 by the addition of an inorganic acid, the surface modified titanium dioxide loses its surface negative charge, resulting in agglomeration. On the other hand, the hydrophilic polymer not bonded to the particles stay in the dispersion liquid without agglomeration so that the hydrophilic polymer remaining unbonded can be removed by centrifugation of this solution.

In the case where precipitation utilizing salting-out is carried out, after the completion of the reaction, the reaction solution is collected in a separatory funnel, an organic solvent for layer separation from water is added, and the mixture is stirred. Upon the completion of the layer separation, the water layer contains surface modified titanium dioxide, while the organic solvent layer contains the aprotic organic solvent used in the reaction. After the separation of the water layer, salt concentration is increased followed by the addition of a suitable amount of polymer such as polyethylene glycol, precipitation of surface modified titanium dioxide occurs by salting-out. This solution is centrifuged to remove the supernatant. Thus, surface modified titanium dioxide fine particles are prepared.

Next, in step (6), the precipitated surface modified titanium dioxide fine particles are washed with water, and the surface modified titanium dioxide fine particles are then suspended in an aqueous solvent having a pH value of 3 to 13, more preferably 5 to 12. As the aqueous solvent, water, a buffer solution having desired pH, or an alkaline aqueous solution can be suitably utilized. The surface modified titanium dioxide fine particles are homogeneously dispersed by stirring this suspension or by application of ultrasonication. The suspension is subjected to desalting and is then dried to give dried powder of surface modified titanium dioxide fine particles. The production of powder which is easy to handle and is stable is very advantageous in using the surface modified titanium dioxide fine particles in various applications.

In the case of composite titanium dioxide fine particles comprising titanium dioxide and a magnetic material, when titanium dioxide is exposed on the surface of the fine particles, the properties of the surface modified titanium dioxide fine particles in the solvent resemble those of the titanium dioxide per se. Therefore, the same production and purification method as described above can be applied. The surface modified composite titanium dioxide fine particles are very advantageous in that, by virtue of magnetic properties of the surface modified composite titanium dioxide fine particles, for example, in applying degradation treatment of harmful substances in water, the fine particles can easily recovered by taking advantage of a magnet after the treatment.

The following Examples further illustrate the present invention but do not limit the present invention.

### Example 1

### Introduction of polyacrylic acid into titanium dioxide particles (1)

Titanium tetraisopropoxide (3.6 g) and 3.6 g of isopropanol were mixed together, and the mixture was added dropwise to 60 ml of ultrapure water under ice cooling for hydrolysis. After the completion of the dropwise addition, the reaction solution was stirred at room temperature for 30 min. After the stirring, 1 ml of 12 N nitric acid was added dropwise thereto, and the mixture was stirred at 80°C for 8 hr for peptization. After the completion of the peptization, the reaction solution was filtered through a 0.45-µm filter, followed by solution exchange through a desalination column (PD10; Amersham Biosciences K.K.) to prepare a titanium dioxide sol having a solid content of 1 %. This dispersion liquid was placed in a 100 ml-volume vial bottle and was ultrasonicated at 200 kHz for 30 min. The average diameter of the dispersed particles before the ultrasonication and the average diameter of the dispersed particles after the ultrasonication were 36.4 nm and 20.2 nm, respectively. After the completion of the ultrasonication, the solution was concentrated to prepare a titanium dioxide sol having a solid content of 20%. The titanium dioxide sol (0.75 ml) thus obtained was dispersed in 20 ml of dimethylformamide (DMF). Polyacrylic acid (average molecular weight: 5000, Wako Pure Chemical Industries, Ltd.) (0.3 g) dissolved in 10 ml of DMF was added to the dispersion liquid, followed by stirring for mixing. The solution was transferred to a hydrothermal reaction vessel (HU-50, SAN-Al Science Co. Ltd.), and synthesis was allowed to proceed at 180°C for 6 hr. After the completion of the reaction, the reaction vessel was cooled to 50°C or below. The solution was taken out of the reaction vessel, 120 ml of water was then added to the solution, followed by stirring for mixing. DMF and water were removed by an evaporator. Thereafter, 20 ml of water was again added to the residue to prepare an aqueous polyacrylic acid-bonded titanium dioxide solution. 2 N hydrochloric acid (1 ml) was added to the aqueous solution to precipitate polyacrylic acid-bonded titanium dioxide fine particles, and the mixture was centrifuged. The supernatant was then removed to separate polyacrylic acid remaining unreacted. Water was again added for washing, the mixture was centrifuged, and water was then removed. A 50 mM phosphate buffer solution (pH 7.0) (10 ml) was added thereto, and the mixture was then ultrasonicated at 200 Hz for 30 min to disperse the polyacrylic acid-bonded titanium dioxide fine particles. After the completion of the ultrasonication, the dispersion liquid was filtered through a 0.45-µm filter to prepare an aqueous polyacrylic acid-bonded titanium dioxide solution having a solid content of 1.5%. The diameter of the dispersed polyacrylic acid-bonded titanium dioxide fine particles thus obtained was measured and was found to be 45.9 nm. The aqueous polyacrylic acid-bonded titanium dioxide solution was desalinated through a desalination column PD10 and was then dried at 100°C to prepare polyacrylic acid-bonded titanium dioxide fine particles (anatase form).

### Example 2

### Introduction of polyacrylic acid into titanium dioxide particles (2)

In quite the same manner as in Example 1, polyacrylic acid-bonded titanium dioxide fine particles were synthesized and were used to prepare an aqueous polyacrylic acid-bonded titanium dioxide solution having a solid content of 1.5%, except that STS-01 (Ishihara Sangyo Kaisha Ltd., solid content: 20%) as a nitric acid-containing acidic anatase sol was used as the titanium dioxide sol. The diameter of the dispersed polyacrylic acid-bonded titanium dioxide fine particles thus obtained was measured and was found to be 66.6 nm. The aqueous polyacrylic acid-bonded titanium dioxide solution was desalinated through a desalination column PD10 and was then dried at 100°C to prepare polyacrylic acid-bonded titanium dioxide fine particles (anatase form).

### Example 3

### Introduction of polyacrylic acid into titanium dioxide particles (part 3)

In quite the same manner as in Example 2, polyacrylic acid-bonded titanium dioxide fine particles were synthesized and were used to prepare an aqueous polyacrylic acid-bonded titanium dioxide solution having a solid content of 1.5%, except that the synthesis temperature was 220°C. The diameter of the dispersed polyacrylic acid-bonded titanium dioxide fine particles thus obtained was measured and was found to be 66.1 nm. The aqueous polyacrylic acid-bonded titanium dioxide solution was desalinated through a desalination column PD10 and was then dried at 100°C to prepare polyacrylic acid-bonded titanium dioxide fine particles (anatase form).

### Example 4

### Introduction of polyacrylic acid into titanium dioxide particles (4)

In quite the same manner as in Example 2, polyacrylic acid-bonded titanium dioxide fine particles were synthesized and were used to prepare an aqueous polyacrylic acid-bonded titanium dioxide solution having a solid content of 1.5%, except that the synthesis temperature was 130°C. The diameter of the dispersed polyacrylic acid-bonded titanium dioxide fine particles thus obtained was measured and was found to be 67.4 nm. The aqueous polyacrylic acid-bonded titanium dioxide solution was desalinated through a desalination column PD10 and was then dried at 100°C to prepare polyacrylic acid-bonded titanium dioxide fine particles (anatase form).

### Example 5

### Introduction of polyacrylic acid into titanium dioxide particles (5)

In quite the same manner as in Example 2, polyacrylic acid-bonded titanium dioxide fine particles were synthesized and were used to prepare an aqueous polyacrylic acid-bonded titanium dioxide solution having a solid content of 1.5%, except that the synthesis temperature was 80°C. The diameter of the dispersed polyacrylic acid-bonded titanium dioxide fine particles thus obtained was measured and was found to be 67.9 nm. The aqueous polyacrylic acid-bonded titanium dioxide solution was desalinated through a desalination column PD10 and was then dried at 100°C to prepare polyacrylic acid-bonded titanium dioxide fine particles (anatase form).

### Example 6

### Introduction of polyacrylic acid into titanium dioxide particles (6)

Titanium tetraisopropoxide (3.6 g) and 3.6 g of isopropanol were mixed together, and the mixture was added dropwise to 60 ml of ultrapure water under ice cooling for hydrolysis. After the completion of the dropwise addition, the reaction solution was stirred at room temperature for 30 min. After the stirring, 1 ml of 12 N nitric acid was added dropwise thereto, and the mixture was stirred at 80°C for 8 hr for peptization. After the completion of the peptization, the reaction solution was filtered through a 0.45-µm filter, followed by solution exchange through a desalination column PD10 to prepare a titanium dioxide sol having a solid content of 1 %. This dispersion liquid was placed in a 100 ml-volume vial bottle and was ultrasonicated at 200 Hz for 30 min. The average diameter of the dispersed particles before the ultrasonication and the average diameter of the dispersed particles after the ultrasonication were 36.4 nm and 20.2 nm, respectively. After the completion of the ultrasonication, the solution was concentrated to prepare a titanium dioxide sol having a solid content of 20%. The titanium dioxide sol (0.75 ml) thus obtained was dispersed in 20 ml of dimethylformamide (DMF). Polyacrylic acid (average molecular weight: 5000, Wako Pure Chemical Industries, Ltd.) (0.3 g) dissolved in 10 ml of DMF was added to the dispersion liquid, followed by stirring for mixing. The Solution was transferred to a hydrothermal reaction vessel (HU-50, SAN-Al Science Co. Ltd.), and synthesis was allowed to proceed at 180°C for 6 hr. After the completion of the reaction, the reaction vessel was cooled to 50°C or below. The solution was taken out of the reaction vessel and was placed in a separatory funnel, 10 ml of water was then added thereto, followed by stirring for mixing. Next, 40 ml of chloroform was added to and mixed with the mixture while stirring, and the lower layer was then removed to recover the upper layer. This step was repeated twice to remove DMF. To 10 ml of this solution were added 10 ml of 1.5 M NaCl and 20% (w/v) polyethylene-glycol 6000 (Wako Pure Chemical Industries, Ltd.). The mixture was centrifuged, and the supernatant was removed. Water (2.5 ml) was added to the precipitate, and the mixture was subjected to gel filtration through a Sephadex G-25 column (Amersham Biosciences K.K.) to prepare a dispersion liquid of polyacrylic acid-bonded titanium dioxide fine particles (anatase form).

### (Example 7)

### Introduction of polyacrylic acid into titanium dioxide particles (7)

Titanium tetraisopropoxide (3.6 g) and 3.6 g of isopropanol were mixed together, and the mixture was added dropwise to 60 ml of ultrapure water under ice cooling for hydrolysis. After the completion of the dropwise addition, the reaction solution was stirred at room temperature for 30 min. After the stirring, 1 ml of 12 N nitric acid was added dropwise thereto, and the mixture was stirred at 80°C for 8 hr for peptization. After the completion of the peptization, the reaction solution was filtered through a 0.45-µm filter, followed by solution exchange through a desalination column PD10 to prepare a titanium dioxide sol having a solid content of 1 %. This dispersion liquid was placed in a 100 ml-volume vial bottle and was ultrasonicated at 200 Hz for 30 min. The average diameter of the dispersed particles before the ultrasonication and the average diameter of the dispersed particles after the ultrasonication were 36.4 nm and 20.2 nm, respectively. After the completion of the ultrasonication, the solution was concentrated to prepare a titanium dioxide sol having a solid content of 20%. The titanium dioxide sol (0.75 ml) thus obtained was dispersed in 20 ml of dimethylformamide (DMF). Polyacrylic acid (average molecular weight: 5000, Wako Pure Chemical industries, Ltd.) (0.3 g) dissolved in 10 ml of DMF was added to the dispersion liquid, followed by stirring for mixing. The solution was transferred to a hydrothermal reaction vessel (HU-50, SAN-Al Science Co. Ltd.), and synthesis was allowed to proceed at 150°C for 5 hr. After the completion of the reaction, the reaction vessel was cooled to 50°C or below. Isopropanol (Wako Pure Chemical Industries, Ltd.) in an amount of twice the amount of the reaction solution was added to the reaction solution. The mixture was left to stand at room temperature for 30 min, and the precipitant was collected by centrifugation. The collected precipitate was washed with 70% ethanol, and 2.5 ml of water was then added to prepare a dispersion liquid of polyacrylic acid-bonded titanium dioxide fine particles (anatase form).

### Example 8

### Introduction of polyacrylic acid into titanium dioxide particles (8)

In quite the same manner as in Example 7, polyacrylic acid-bonded titanium dioxide fine particles were synthesized, except that polyacrylic acid having an average molecular weight of 2000 and polyacrylic acid having an average molecular weight of 3500 were used. In both the cases where polyacrylic acid having an average molecular weight of 2000 and polyacrylic acid having an average molecular weight of 3500 were used, the dispersion liquids of polyacrylic acid-bonded titanium dioxide fine particles (anatase form) were good and had good dispersibility.

### Example 9

### Introduction of polyacrylic acid into magnetic material/titanium oxide composite fine particles

Polyoxyethylene (15) cetyl ether (C-15: NIHON SURFACTANT KOGYO K.K.) (45.16 g) was dissolved in a separable flask, and the air in the flask was replaced by nitrogen for 5 min. Cyclohexene (Wako Pure Chemical Industries, Ltd.) (75 ml) was added to the solution, a 0.67 M aqueous FeCl₂ (Wako Pure Chemical Industries, Ltd.) solution (3.6 ml) was added, 5.4 ml of a 30% aqueous ammonia solution was then added thereto with stirring at 250 rpm, and a reaction was allowed to proceed for one hr. Thereafter, 0.4 ml of a 50 mM aqueous tetraethylorthosilicate solution (Wako Pure Chemical Industries, Ltd.) was added dropwise thereto, and a reaction was allowed to proceed for one hr. Thereafter, titanium tetraisopropoxide (Wako Pure chemical Industries, Ltd.) was added to a final concentration of 5 mM. A 50% (w/v) aqueous ethanol solution (10 ml) was added in 1 ml portions at intervals of 10 min. The aqueous solution was centrifuged, and the precipitate was fired at 350°C for 2 hr. After the completion of the firing, the fired product was dispersed in a 10 mM aqueous nitric acid solution, and the dispersion liquid was ultrasonicated, followed by filtration through a 0.1-µm filter. The magnetic material/titanium oxide composite sol (0.75 ml) thus obtained was dispersed in 20 ml of dimethylformamide (DMF), and a solution of 0.3 g of polyacrylic acid (average molecular weight: 5000, Wako Pure Chemical Industries, Ltd.) dissolved in 10 ml of DMF was added to the dispersion liquid, followed by stirring for mixing. The solution was transferred to a hydrothermal reaction vessel (HU-50, SAN-Al Science Co. Ltd.), and synthesis was allowed to proceed at 180°C for 6 hr. After the completion of the reaction, the reaction vessel was cooled to 50°C or below. The solution was taken out of the reaction vessel and was placed in a separatory funnel, 10 ml of water was then added thereto, followed by stirring for mixing. Next, 40 ml of chloroform was added to and mixed with the mixture while stirring, and the lower layer was then removed to recover the upper layer. This step was repeated twice to remove DMF. To 10 ml of this solution were added 10 ml of 1.5 M NaCl and 20% (w/v) polyethylene-glycol 6000 (Wako Pure Chemical Industries, Ltd.). The mixture was centrifuged, and the supernatant was then removed. Water (2.5 ml) was added to the precipitate, and the mixture was subjected to gel filtration through a Sephadex G-25 column (Amersham Biosciences K.K.) to prepare a dispersion liquid of polyacrylic acid-bonded magnetic material/titanium dioxide composite fine particles (anatase form). This dispersion liquid was not cloudy and contained well dispersed fine particles, that is, was a good dispersion liquid, as with the case of single titanium dioxide.

### Example 10

### Introduction of acrylic acid/sulfonic acid copolymer into titanium dioxide particles

The titanium dioxide sol having a solid content of 20% (0.75 ml) produced in the process of Example 7 was dispersed in 10 ml of DMF. An acrylic acid/sulfonic acid monomer copolymer (GL 386, manufactured by Nippon Shokubai Kagaku Kogyo Co., Ltd.; average molecular weight: 5000; a preparation obtained by replacing sodium with proton by cation exchange resin and then conducting lyophilization) (0.3 g) dissolved in 10 ml of DMF was added to the dispersion liquid, followed by mixing with stirring. The mixed liquid was transferred to a hydrothermal reaction vessel (HU-50, SAN-AI Science Co. Ltd.), and synthesis was allowed to proceed at 150°C for 5 hr. After the completion of the reaction, the reaction vessel was cooled to room temperature. Isopropanol (Wako Pure Chemical Industries, Ltd.) in an amount of twice the amount of the reaction solution was added to the reaction solution. The mixture was left to stand at room temperature for 30 min, and the precipitant was then collected by centrifugation. The collected precipitate was washed with 70% ethanol, and 2.5 ml of water was then added to prepare a dispersion liquid of an acrylic acid/sulfonic acid copolymer-bonded titanium dioxide fine particles (anatase form). This dispersion liquid was not cloudy and contained well dispersed fine particles and, as with the case of polyacrylic acid, was good.

### Example 11

### Effect of polyacrylic acid concentration on dispersibility of polyacrylic acid-bonded titanium dioxide fine particles

The titanium dioxide sol having a solid content of 20% (0.75 ml) produced in the process of Example 7 was dispersed in 10 ml of DMF. DMF solutions (5 ml) containing polyacrylic acid (average molecular weight: 5000, Wako Pure Chemical Industries, Ltd.) with varied weight levels were added to the dispersion liquid, followed by mixing with stirring. The solution was transferred to a hydrothermal reaction vessel (HU-50, SAN-AI Science Co. Ltd.), and synthesis was allowed to proceed at 150°C for 5 hr. After the completion of the reaction, the properties of the individual solutions were observed. As a result, it was found that, when the final concentration of polyacrylic acid was not less than 0.4 mg/ml, dispersion liquids containing well dispersed polyacrylic acid-bonded titanium dioxide fine particles were provided, whereas, when the concentration of polyacrylic acid was less than 0.4 mg/ml, although the particles were dispersed, the dispersion liquids were cloudy and had large particle diameters, indicating that, under the above reaction conditions, the final concentration of polyacrylic acid should be not less than 0.4 mg/ml

### Example 12

### Effect of water content on dispersibility of polyacrylic acid-bonded titanium dioxide fine particles

The titanium dioxide sol having a solid content of 20% (0.75 ml) produced in the process of Example 7 was dispersed in 10 ml of DMF. DMF solutions (5 ml) containing 30 mg/ml polyacrylic acid (average molecular weight: 5000, Wako Pure Chemical Industries, Ltd.) with varied water content levels were added to the dispersion liquid, followed by mixing with stirring. The mixed liquid was transferred to a hydrothermal reaction vessel (HU-50, SAN-Al Science Co. Ltd.), and synthesis was allowed to proceed at 150°C for 5 hr. After the completion of the reaction, the properties of the individual solutions were observed. As a result, it was found that, when the final water content was not more than 4%, dispersion liquids containing well dispersed polyacrylic acid-bonded titanium dioxide fine particles were provided, whereas, when the final water content was not less than 5%, although the particles were dispersed, the dispersion liquids were cloudy and had large particle diameters, indicating that, under the above reaction conditions, the water content at the time of the reaction is preferably not more than 4% in terms of final concentration.

### Example 13

### Solubility of polyacrylic acid-bonded titanium dioxide fine particles in isopropanol

A solution of 1 g of polyacrylic acid in 10 ml of DMF was designated as solution (A). A dispersion liquid of 0.25 ml of the titanium dioxide sol having a solid content of 20% produced in the process of Example 7 in 10 ml of DMF was designated as solution (B). Further, a dispersion liquid of 0.25 ml of the titanium dioxide sol having a solid content of 20% produced in the process of Example 7 and 1 g of 20% (w/v) polyacrylic acid in 10 ml of DMF was designated as solution (C). Furthermore, a dispersion liquid of polyacrylic acid-bonded titanium dioxide fine particles prepared by reacting solution (C) at 150°C for 5 hr was designated as solution (D). To each of solutions (A) to (D) was added isopropanol in an amount of twice the amount of each solution. The mixtures were stirred and were then left to stand for inspection of precipitate formation. As a result, it was found that all of solutions (A) to (C) were soluble in isopropanol, and only solution (D) caused precipitate formation. This indicates that all of polyacrylic acid (A), titanium dioxide sol (B), mixed liquid (C) composed of polyacrylic acid and titanium dioxide sol are soluble in isopropanol, whereas polyacrylic acid-bonded titanium dioxide fine particles (D) is insoluble. Solution (C) will be compared with solution (D). In the system (C) in which polyacrylic acid as a dispersant had been merely added to titanium dioxide sol, it is considered that both the substances are not chemically reacted with each other and each independently are soluble in isopropanol. On the other hand, in solution (D), it is considered that titanium dioxide and polyacrylic acid are bonded to each other by a hydration reaction through an ester linkage and, due to hydrophilicity of an infinite number of carboxyl residues of polyacrylic acid bonded to the surface of titanium oxide fine particles, this solution is insoluble in isopropanol.

### Example 14

### Stability of polyacrylic acid-bonded titanium dioxide fine particles in neutral solution

Solutions respectively having the same compositions as solutions (A) to (D) used in Example 13 were evaluated for stability in a neutral solution. Specifically, each of solutions (A) to (D) was diluted with a 200 mM phosphate buffer (pH 7.0) by a factor of 10, followed by stirring. The diluted solutions were then left to stand and were observed for precipitate formation. As a result, for solutions (B) and (C) each containing titanium dioxide sol, precipitate formation was observed, whereas, for solutions (A) and (D), precipitate formation was not observed. This difference is believed to reside in that, since the isoelectric point of titanium dioxide exists in a near neutral state, for solutions (B) and (C), titanium dioxide was agglomerated resulting in precipitate formation. On the other hand, for solution (D), since the surface of titanium dioxide is modified with an infinite number of carboxyl residues, the isoelectric point of the whole particles exist in a pH value around 2.8 and, thus, a homogeneously dispersed state is held even in a neutral solution. That is, a comparison of solution (C) with solution (D) based on the results of Example 13 and Example 14 showed that polyacrylic acid-bonded titanium dioxide fine particles exhibit properties which are utterly different from properties of titanium dioxide fine particles having dispersibility enhanced by merely adding polyacrylic acid.

### Example 15

### Evaluation of polyacrylic acid-bonded titanium dioxide fine particles for pH stability

The polyacrylic acid-bonded titanium dioxide fine particles prepared in Examples 1 to 7 were dispersed in water, and the pH value of the solutions were varied from pH 3 to pH 13 using hydrochloric acid and sodium hydroxide in an increment of pH 1, and the solutions were observed for agglomeration or precipitation formation of the polyacrylic acid-bonded titanium dioxide fine particles. The aqueous solutions with varied pH were centrifuged at 4000 rpm and were observed for agglomeration. As a result, it was found that, in all pH values, neither agglomeration nor precipitation of the particles was observed.

### Example 16

### Infrared spectroscopic (FT-IR) analysis of polyacrylic acid-bonded titanium dioxide fine particles

A dispersion liquid of purified polyacrylic acid-bonded titanium dioxide fine particles with free polyacrylic acid completely removed therefrom was lyophilized and were used for preparation of KBr tablets by a conventional method. FT-IR was measured with an infrared spectrophotometer (FTS-65A, Bio-Rad Laboratories, Inc.). Characteristic absorptions of polyacrylic acid-bonded titanium dioxide fine particles are shown in Table 1. In Table 1, for example, a) large and broad absorption attributable to stretching vibration of intermolecular hydrogen-bonded O-H is observed at 2500 to 3500 cm⁻¹; b) absorption around 2900 cm⁻¹ attributable to stretching vibration of methylene, which is not observed for titanium dioxide, is observed; c) absorption attributable to C=O stretching vibration of an ester linkage, which is not found in titanium dioxide, is observed at 1720 cm⁻¹; and d) absorption attributable to deformation vibration derived from polyacrylic acid, which is not found in titanium dioxide, is observed on low wavenumber side. These facts reveal that, in the synthesized polyacrylic acid-bonded titanium dioxide fine particles, titanium dioxide and polyacrylic acid have been certainty chemically bonded to each other through an ester linkage. An absorption peak attributable to C=O stretching vibration of carboxylic acid appears on lower wavenumber side than that for ester and thus appears to overlap with a large absorption peak derived from titanium dioxide at 1650 cm⁻¹. On the other hand, an FT-IR spectrum of a product produced by completely removing polyacrylic acid from a system, in which polyacrylic acid was merely added as a dispersant to titanium dioxide sol without conducting any hydrothermal reaction followed by lyophilization, was substantially in agreement with a spectrum of titanium dioxide per se. That is, the results of FT-IR analysis for Examples 13 to 15 and this Example have revealed that, in the polyacrylic acid-bonded titanium dioxide fine particles according to the present invention, titanium dioxide and polyacrylic acid are strongly bonded to each other through an ester linkage, whereas, in the case where polyacrylic acid as a dispersant was merely added to titanium dioxide, titanium dioxide and polyacrylic acid are not chemically bonded to each other and the effect attained in this case is merely an improvement in dispersibility through electrostatic interaction.

**Table 1: Infrared spectroscopic analysis of polyacrylic acid-bonded titanium dioxide fine particles**

| Absorption wave number cm⁻¹ | | | | | | | | | |
|---|---|---|---|---|---|---|---|---|---|
| -3386- | 2931 | 1720 | 1650 | 1454 | 1393 | 1256 | 1171 | 1106 | 1059 |
| O-H stretching (broad) | C-H stretching | C=O stretching, ester bond | Derived from titanium dioxide | C-H deformation | Derived from titanium dioxide | Derived from polyacrylic acid | Derived from polyacrylic acid | Derived from polyacrylic acid | Derived from polyacrylic acid |

### Example 17

### Determination of content of titanium dioxide in dispersion liquid of polyacrylic acid-bonded titanium dioxide fine particles

The dispersion liquid of the polyacrylic acid-bonded titanium dioxide fine particles prepared in Example 7 was heat dried at 110°C for one hr and was further ignited for 4 hr for complete incineration. The ash was cooled in a silica gel desiccator, and the mass was measured to determine the net amount of titanium dioxide in the dispersion liquid. As a result, it was found that the dispersion liquid contained 8.82% (w/v) of titanium dioxide.

### Example 18

### Determination of content of carboxylic acid in dispersion liquid of polyacrylic acid-bonded titanium dioxide fine particles

Water (100 ml) and 20 ml of a 0.1 M NaOH standard solution were added to 1 ml of the dispersion liquid of the polyacrylic acid-bonded titanium dioxide fine particles prepared in Example 7. After thorough mixing with stirring, the remaining NaOH was back-titrated with a 0.1 M HCl standard solution to determine the carboxyl group content of the polyacrylic acid-bonded titanium dioxide fine particles. As a result, it was found that 1 ml of the dispersion liquid contained 3.08 × 10⁻⁴ mol of carboxyl group. From the results of Examples 16 and 17, it was found that the content ratio of carboxyl group/titanium dioxide in the dispersion liquid was 3.08 × 10⁻² (mol/g). Likewise, for a dispersion liquid of the polyacrylic acid-bonded titanium dioxide fine particles synthesized using polyacrylic acid having a molecular weight of 2000 in Example 8, the content ratio of carboxyl group/titanium dioxide in the dispersion liquid was 2.64 × 10⁻² (mol/g). As a result of further studies, it was found that, in order to ensure uniform dispersibility in the dispersion liquid of polyacrylic acid-bonded titanium dioxide fine particles, the content ratio of carboxyl group/titanium dioxide is preferably not less than 2 × 10⁻³ (mol/g).

### Example 19

### Evaluation of polyacrylic acid-bonded titanium dioxide fine particles (anatase form) for photocatalytic activity

The polyacrylic acid-bonded titanium dioxide fine particles (anatase form) prepared in Examples 1 to 5 were diluted with a 50 mM phosphate buffer (pH 7.0) to a solid content of 0.02%. Methylene blue trihydrate (Wako Pure Chemical Industries, Ltd.) was added to the aqueous solution to a concentration of 40 µM. This aqueous solution was exposed with stirring to ultraviolet light with a wavelength of 340 nm at 1.5 mW/cm², and wavelength absorption at 580 nm was measured with an ultraviolet-visible light spectrophotometer. The results are shown in Fig. 2. For all the samples, a reduction in absorbance derived from degradation of methylene blue with the elapse of ultraviolet light irradiation time was observed, indicating that the polyacrylic acid-bonded titanium dioxide fine particles (anatase form) prepared in Examples 1 to 5 had photocatalytic activity.

### Example 20

### Evaluation of antimicrobial activity of polyacrylic acid-bonded titanium dioxide fine particles (anatase form)

A 50 mM phosphate buffer (pH 7.0) was added to the polyacrylic acid-bonded titanium dioxide fine particles (anatase form) prepared in Example 1 to a solid content of 1.0%. Escherichia coli was cultured in LB broth at 37°C overnight. The culture was then centrifuged, and the cells were rinsed with a 50 mM phosphate buffer (pH 7.0) and were suspended in a 50 mM phosphate buffer (pH 7.0) in an amount equal to the amount of the culture. This was further diluted with a 50 mM phosphate buffer (pH 7.0) by a factor of 100 to prepare a test bacterial suspension. The above polyacrylic acid-bonded titanium dioxide fine particles were added to the test bacterial suspension to a final concentration of 0.1 %. The mixed liquid composed of the test bacterial suspension and the polyacrylic acid-bonded titanium dioxide fine particles was placed in a small-size Schale to a liquid depth of 3 mm and was left to stand at room temperature under blacklight irradiation (dose: 900 µW/cm²). Further, the test bacterial suspension with no polyacrylic acid-bonded titanium dioxide fine particles added thereto was provided as control 1 and was exposed to blacklight in the same manner as described above. Before irradiation, 2 hr after irradiation, and 4 hr after irradiation, a part of each of the test liquids was collected, and cell counting was carried out by a conventional method using an LB agar medium. Separately, the polyacrylic acid-bonded titanium dioxide fine particles were added to the test lysate to a final concentration of 0.1%, and the mixture was left to stand under light shielding conditions. This sample was designated as control 2. When the same times as described above elapsed, cell counting was carried out. The results are shown in Table 2. In the system in which polyacrylic acid-bonded titanium dioxide fine particles (anatase form) were added followed by blacklight irradiation, the cell count decreased with the elapse of time, indicating that the fine particles had antimicrobial activity associated with photocatalytic activity.

**Table 2: Evaluation of polyacrylic acid-bonded titanium dioxide fine particles for antimicrobial activity (CFS/ml)**

| | Fine particles of present invention, solid content 0.1% | Control 1 | Control 2 |
|---|---|---|---|
| Before irradiation | 4.2 × 10⁷ | 4.0 × 10⁷ | 4.1 × 10⁷ |
| After 2 hr | 5.6 × 10⁶ | 3.5 × 10⁷ | 4.0 × 10⁷ |
| After 4 hr | 2.6 × 10⁴ | 3.5 × 10⁷ | 3.8 × 10⁷ |

### Example 21

### Evaluation of cytotoxic activity against cancer cells

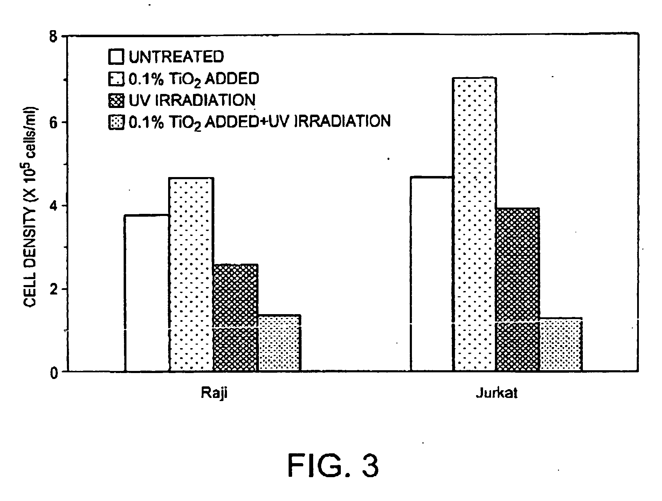

PBS buffer (pH 6.8) was added to polyacrylic acid-bonded titanium dioxide fine particles (anatase form) prepared in Example 1 to a solid content of 1.0%. Two types of cultured cancer cells (Raji, Jurkat) were cultured in an RPMI 1640 medium containing 10% serum (manufactured by Gibco) at 37°C under a 5% carbon dioxide atmosphere to prepare 5.8 × 10⁵ cell fluid. This was again cultured under the same conditions for 20 hr to prepare a test cell fluid. The above dispersion liquid of polyacrylic acid-bonded titanium dioxide fine particles was added to this test cell fluid to a final concentration of 0.1% to prepare a test liquid. This test liquid was placed in a small-size Schale to a liquid depth of 3 mm, and the Schale was then left to stand at room temperature under blacklight (UV) irradiation (dose: 900 µW/cm²). In this case, a blank (untreated) in which neither the addition of polyacrylic acid-bonded titanium dioxide fine particles to the test liquid nor UV irradiation was carried out, a control in which UV irradiation was not carried out after the addition of polyacrylic acid-bonded titanium dioxide fine particles, and a control in which UV was merely applied to the test liquid without the addition of the polyacrylic acid-bonded titanium dioxide fine particles, were also simultaneously tested. A sample of each test liquid was collected 6 hr after the initiation of the test, and cell counting was carried out. The results are shown in Fig. 3. In the system in which the polyacrylic acid-bonded titanium dioxide fine particles were added and UV irradiation was also carried out, a marked reduction in cell count was observed, indicating that the polyacrylic acid-bonded titanium dioxide fine particles had cytotoxic activity against cancer cells.

### INDUSTRIAL APPLICABILITY

According to the present invention, there are provided surface modified titanium dioxide fine particles, which are suitable for use in various applications, are excellent in dispersibility in neutral aqueous solvents, and have stable dispersibility for a long period of time in a broad range of pH, a dispersion liquid of the surface modified titanium dioxide fine particles, and a process for producing the surface modified titanium dioxide fine particles.

## Claims

1. Surface modified titanium dioxide fine particles comprising titanium dioxide having a surface which is modified with a hydrophilic polymer having carboxyl groups, the carboxyl groups in the hydrophilic polymer being bonded to titanium dioxide through an ester linkage, wherein said titanium dioxide is an anatase or rutile form of titanium dioxide.

2. The surface modified titanium dioxide fine particles according to claim 1, wherein said titanium dioxide has a particle diameter of 2 to 200 nm.

3. The surface modified titanium dioxide fine particles according to claim 1 or 2, wherein said titanium dioxide is a composite titanium dioxide comprising titanium dioxide and a magnetic material.

4. The surface modified titanium dioxide fine particles according to any one of claims 1 to 3, wherein said hydrophilic polymer is a water soluble polymer.

5. The surface modified titanium dioxide fine particles according to claim 4, wherein said water soluble polymer contains a polycarboxylic acid.

6. The surface modified titanium dioxide fine particles according to claim 4, wherein said water soluble polymer comprises a copolymer having a plurality of carboxyl group units in its molecule.

7. A dispersion liquid of surface modified titanium dioxide fine particles, comprising the surface modified titanium dioxide fine particles according to any one of claims 1 to 6 dispersed in an aqueous solvent.

8. The dispersion liquid of surface modified titanium dioxide fine particles according to claim 7, wherein said aqueous solvent has a pH value of 3 to 13.

9. The dispersion liquid of surface modified titanium dioxide fine particles according to claim 8, wherein said aqueous solvent is a pH buffer solution.

10. The dispersion liquid of surface modified titanium dioxide fine particles according to claim 8, wherein said aqueous solvent is physiological saline.

11. The dispersion liquid of surface modified titanium dioxide fine particles according to any one of claims 8 to 10, for use as an auxiliary material for phototherapy in which the auxiliary material is introduced into the body in its affected region and light such as ultraviolet light is then applied to the affected region to destroy the affected region.

12. The dispersion liquid of surface modified titanium dioxide fine particles according to claim 11, wherein said affected region is a cancer tissue.

13. A process for producing surface modified titanium dioxide fine particles by chemically bonding a hydrophilic polymer to the surface of titanium dioxide fine particles, wherein said titanium dioxide is an anatase or rutile form of titanium dioxide said process comprising: (1) the first step of dispersing a titanium dioxide sol in a solvent; (2) the second step of dispersing a hydrophilic polymer in a solvent; (3) the third step of mixing the two dispersion liquids together; (4) the fourth step of heating the mixed liquid; (5) the fifth step of separating the surface modified titanium dioxide fine particles from the hydrophilic polymer remaining unbonded; and (6) the sixth step of purifying the surface modified titanium dioxide fine particles.

14. The process for producing surface modified titanium dioxide fine particles according to claim 13, wherein the solvent used in the first step and the solvent used in the second step are an aprotic solvent.

15. The process for producing surface modified titanium dioxide fine particles according to claim 14, wherein said aprotic solvent is any of dimethylformamide, dioxane, and dimethylsulfoxide.

16. The process for producing surface modified titanium dioxide fine particles according to any one of claims 13 to 15, wherein the heating temperature in the fourth step is 80 to 220 deg. C.

17. The process for producing surface modified titanium dioxide fine particles according to any one of claims 13 to 16, wherein the fifth step for the separation comprises the step of adjusting the mixed liquid to pH not more than 2.8 to allow only the surface modified titanium dioxide fine particles to cause isoelectric coagulation, whereby the hydrophilic polymer remaining unbonded as the supernatant is removed.

18. The process for producing surface modified titanium dioxide fine particles according to any one of claims 13 to 16, wherein the fifth step for the separation comprises the step of removing the hydrophilic molecules remaining unbonded by molecular sieves.

19. The process for producing surface modified titanium dioxide fine particles according to any one of claims 13 to 18, wherein the sixth step for the purification comprises the step of dispersing the surface modified titanium dioxide fine particles in an aqueous solvent and then drying the fine particles.

20. The process for producing surface modified titanium dioxide fine particles according to any one of claims 13 to 18, wherein the fifth step for the separation comprises the step of dispersing the surface modified titanium dioxide fine particles in an aqueous solvent and then precipitating the surface modified titanium dioxide fine particles by salting-out.

21. The process for producing surface modified titanium dioxide fine particles according to any one of claims 13 to 18, wherein the fifth step for the separation comprises the step of dispersing the surface modified titanium dioxide fine particles in an aqueous solvent and then precipitating the surface modified titanium dioxide fine particles from an organic solvent.

## Patentansprüche

1. oberflächenmodifizierte Titandioxid-Feinpartikel, die Titandioxid mit einer Oberfläche umfassen, die mit einem hydrophilen Polymer mit Carboxylgruppen modifiziert ist, wobei die Carboxylgruppen in dem hydrophilen Polymer durch eine Esterbindung an Titandioxid gebunden sind, wobei das Titandioxid eine Anatas- oder Rutilform von Titandioxid ist.

2. Oberflächenmodifizierte Titandioxid-Feinpartikel nach Anspruch 1, wobei das Titandioxid einen Partikeldurchmesser von 2 bis 200 nm aufweist.

3. Oberflächenmodifizierte Titandioxid-Feinpartikel nach Anspruch 1 oder 2, wobei das Titandioxid ein Verbundtitandioxid ist, das Titandioxid und ein magnetisches Material umfasst.

4. Oberflächenmodifizierte Titandioxid-Feinpartikel nach einem der Ansprüche 1 bis 3, wobei das hydrophile Polymer ein wasserlösliches Polymer ist.

5. Oberflächenmodifizierte Titandioxid-Feinpartikel nach Anspruch 4, wobei das wasserlösliche Polymer eine Polycarbonsäure enthält.

6. Oberflächenmodifizierte Titandioxid-Feinpartikel nach Anspruch 4, wobei das wasserlösliche Polymer ein Copolymer mit mehreren Carboxylgruppeneinheiten in seinem Molekül umfasst.

7. Dispersionsflüssigkeit von oberflächenmodifizierten Titandioxid-Feinpartikeln, die die oberflächenmodifizierten Titandioxid-Feinpartikel nach einem der Ansprüche 1 bis 6 in einem wässrigen Lösungsmittel dispergiert umfasst.

8. Dispersionsflüssigkeit von oberflächenmodifizierten Titandioxid-Feinpartikeln nach Anspruch 7, wobei das wässrige Lösungsmittel einen pH-Wert von 3 bis 13 aufweist.

9. Dispersionsflüssigkeit von oberflächenmodifizierten Titandioxid-Feinpartikeln nach Anspruch 8, wobei das wässrige Lösungsmittel eine pH-Pufferlösung ist.

10. Dispersionsflüssigkeit von oberflächenmodifizierten Titandioxid-Feinpartikeln nach Anspruch 8, wobei das wässrige Lösungsmittel physiologische Kochsalzlösung ist,

11. Dispersionsflüssigkeit von oberflächenmodifizierten Titandioxid-Feinpartikeln nach einem der Ansprüche 8 bis 10 zur Verwendung als ein Hilfsmaterial für die Phototherapie, wobei das Hilfsmaterial in den Körper in dessen betroffene Region eingebracht wird und Licht, wie ultraviolettes Licht, dann auf die betroffene Region angewendet wird, um die betroffene Region zu zerstören.

12. Dispersionsflüssigkeit von oberflächenmodifizierten Titandioxid-Feinpartikeln nach Anspruch 11, wobei die betroffene Region ein Krebsgewebe ist.

13. Verfahren zur Herstellung von oberflächenmodifizierten Titandioxid-Feinpartikeln durch chemisches Binden eines hydrophilen Polymers an die Oberfläche von Titandioxid-Feinpartikeln, wobei das Titandioxid eine Anatas- oder Rutilform von Titandioxid ist, wobei das Verfahren Folgendes umfasst: (1) den ersten Schritt des Dispergierens eines Titandioxid-Sols in einem Lösungsmittel; (2) den zweiten Schritt des Dispergierens eines hydrophilen Polymers in einem Lösungsmittel; (3) den dritten Schritt des Mischens der zwei Dispersionsflüssigkeiten miteinander; (4) den vierten Schritt des Erhitzens der Mischflüssigkeit; (5) den fünften Schritt des Trennens der oberflächenmodifizierten Titandioxid-Feinpartikel von dem hydrophilen Polymer, das ungebunden geblieben ist; und (6) den sechsten Schritt des Aufreinigens der oberflächenmodifizierten Titandioxid-Feinpartikel.

14. Verfahren zur Herstellung von oberflächenmodifizierten Titandioxid-Feinpartikeln nach Anspruch 13, wobei das in dem ersten Schritt verwendete Lösungsmittel und das in dem zweiten Schritt verwendete Lösungsmittel ein aprotisches Lösungsmittel sind.

15. Verfahren zur Herstellung von oberflächenmodifizierten Titandioxid-Feinpartikeln nach Anspruch 14, wobei das aprotische Lösungsmittel ein beliebiges von Dimethylformamid, Dioxan und Dimethylsulfoxid ist.

16. Verfahren zur Herstellung von oberflächenmodifizierten Titandioxid-Feinpartikeln nach einem der Ansprüche 13 bis 15, wobei die Erhitzungstemperatur in dem vierten Schritt 80 bis 220 °C ist.

17. Verfahren zur Herstellung von oberflächenmodifizierten Titandioxid-Feinpartikeln nach einem der Ansprüche 13 bis 16, wobei der fünfte Schritt zur Trennung den Schritt des Einstellens der Mischflüssigkeit auf einen pH-Wert von nicht mehr als 2,8 umfasst, um nur den oberflächenmodifizierten Titandioxid-Feinpartikeln zu ermöglichen, eine isoelektrische Koagulation zu bewirken, wodurch das hydrophile Polymer ungebunden bleibt, wenn der Überstand entfernt wird.

18. Verfahren zur Herstellung von oberflächenmodifizierten Titandioxid-Feinpartikeln nach einem der Ansprüche 13 bis 16, wobei der fünfte Schritt zur Trennung den Schritt des Entfernens der hydrophilen Moleküle, die ungebunden geblieben sind, durch Molekularsiebe umfasst.

19. Verfahren zur Herstellung von oberflächenmodifizierten Titandioxid-Feinpartikeln nach einem der Ansprüche 13 bis 18, wobei der sechste Schritt zur Aufreinigung den Schritt des Dispergierens der oberflächenmodifizierten Titandioxid-Feinpartikel in einem wässrigen Lösungsmittel und dann des Trocknens der Feinpartikel umfasst.

20. Verfahren zur Herstellung von oberflächenmodifizierten Titandioxid-Feinpartikeln nach einem der Ansprüche 13 bis 18, wobei der fünfte Schritt zur Trennung den Schritt des Dispergierens der oberflächenmodifizierten Titandioxid-Feinpartikel in einem wässrigen Lösungsmittel und dann des Ausfällens der oberflächenmodifizierten Titandioxid-Feinpartikel durch Aussalzen umfasst.

21. Verfahren zur Herstellung von oberflächenmodifizierten Titandioxid-Feinpartikeln nach einem der Ansprüche 13 bis 18, wobei der fünfte Schritt zur Trennung den Schritt des Dispergierens der oberflächenmodifizierten Titandioxid-Feinpartikel in einem wässrigen Lösungsmittel und dann des Ausfällens der oberflächenmodifizierten Titandioxid-Feinpartikel aus einem organischen Lösungsmittel umfasst.

## Revendications

1. Fines particules de dioxyde de titane modifiées en surface comprenant du dioxyde de titane ayant une surface qui est modifiée avec un polymère hydrophile ayant des groupes carboxyles, les groupes carboxyles dans le polymère hydrophile étant liés au dioxyde de titane par l'intermédiaire d'une liaison ester, dans lesquelles ledit dioxyde de titane est une anatase ou une forme rutile de dioxyde de titane.

2. Fines particules de dioxyde de titane modifiées en surface selon la revendication 1, dans lesquelles ledit dioxyde de titane a un diamètre de particules de 2 à 200 nm.

3. Fines particules de dioxyde de titane modifiées en surface selon la revendication 1 ou la revendication 2, dans lesquelles ledit dioxyde de titane est un dioxyde de titane composite comprenant du dioxyde de titane et un matériau magnétique.

4. Fines particules de dioxyde de titane modifiées en surface selon l'une quelconque des revendications 1 à 3, dans lesquelles ledit polymère hydrophile est un polymère hydrosoluble.

5. Fines particules de dioxyde de titane modifiées en surface selon la revendication 4, dans lesquelles ledit polymère hydrosoluble contient un acide polycarboxylique.

6. Fines particules de dioxyde de titane modifiées en surface selon la revendication 4, dans lesquelles ledit polymère hydrosoluble comprend un copolymère ayant une pluralité d'unités de groupe carboxyle dans sa molécule.

7. Liquide de dispersion de fines particules de dioxyde de titane modifiées en surface, comprenant les fines particules de dioxyde de titane modifiées en surface selon l'une quelconque des revendications 1 à 6 dispersées dans un solvant aqueux.

8. Liquide de dispersion de fines particules de dioxyde de titane modifiées en surface selon la revendication 7, dans lequel ledit solvant aqueux a une valeur de pH de 3 à 13.

9. Liquide de dispersion de fines particules de dioxyde de titane modifiées en surface selon la revendication 8, dans lequel ledit solvant aqueux est une solution de tampon pour pH.

10. Liquide de dispersion de fines particules de dioxyde de titane modifiées en surface selon la revendication 8, dans lequel ledit solvant aqueux est une solution saline physiologique.

11. Liquide de dispersion de fines particules de dioxyde de titane modifiées en surface selon l'une quelconque des revendications 8 à 10, à utiliser comme un matériau auxiliaire pour une photothérapie dans laquelle le matériau auxiliaire est introduit dans le corps dans sa région affectée et de la lumière telle que de la lumière ultraviolette est ensuite appliquée à la région affectée pour détruire la région affectée.

12. Liquide de dispersion de fines particules de dioxyde de titane modifiées en surface selon la revendication 11, dans lequel ladite région affectée est un tissu cancéreux.

13. Processus de production de fines particules de dioxyde de titane modifiées en surface consistant à lier chimiquement un polymère hydrophile à la surface de fines particules de dioxyde de titane, dans lequel ledit dioxyde de titane est une anatase ou une forme rutile de dioxyde de titane, ledit processus comprenant : (1) la première étape consistant à disperser une solution de dioxyde de titane dans un solvant ; (2) la deuxième étape consistant à disperser un polymère hydrophile dans un solvant ; (3) la troisième étape consistant à mélanger ensemble les deux liquides de dispersion ; (4) la quatrième étape consistant à chauffer le liquide mélangé ; (5) la cinquième étape consistant à séparer les fines particules de dioxyde de titane modifiées en surface du polymère hydrophile restant non lié ; et (6) la sixième étape consistant à purifier les fines particules de dioxyde de titane modifiées en surface.

14. Processus de production de fines particules de dioxyde de titane modifiées en surface selon la revendication 13, dans lequel le solvant utilisé dans la première étape et le solvant utilisé dans la deuxième étape sont un solvant aprotique.

15. Processus de production de fines particules de dioxyde de titane modifiées en surface selon la revendication 14, dans lequel ledit solvant aprotique est un quelconque parmi du diméthylformamide, du dioxane, et du diméthylsulfoxyde.

16. Processus de production de fines particules de dioxyde de titane modifiées en surface selon l'une quelconque des revendications 13 à 15, dans lequel la température de chauffage à la quatrième étape est de 80 à 220 degrés C.

17. Processus de production de fines particules de dioxyde de titane modifiées en surface selon l'une quelconque des revendications 13 à 16, dans lequel la cinquième étape pour la séparation comprend l'étape consistant à ajuster le liquide mélangé à un pH inférieur ou égal à 2,8 pour permettre uniquement aux fines particules de dioxyde de titane modifiées en surface de provoquer une coagulation isoélectrique, par laquelle le polymère hydrophile restant non lié en tant que surnageant est éliminé.

18. Processus de production de fines particules de dioxyde de titane modifiées en surface selon l'une quelconque des revendications 13 à 16, dans lequel la cinquième étape pour la séparation comprend l'étape consistant à éliminer les molécules hydrophiles restant non liées au moyen de tamis moléculaires.

19. Processus de production de fines particules de dioxyde de titane modifiées en surface selon l'une quelconque des revendications 13 à 18, dans lequel la sixième étape pour la purification comprend l'étape consistant à disperser les fines particules de dioxyde de titane modifiées en surface dans un solvant aqueux, puis à sécher les fines particules.

20. Processus de production de fines particules de dioxyde de titane modifiées en surface selon l'une quelconque des revendications 13 à 18, dans lequel la cinquième étape pour la séparation comprend l'étape consistant à disperser les fines particules de dioxyde de titane modifiées en surface dans un solvant aqueux, puis à précipiter les fines particules de dioxyde de titane modifiées en surface par relargage.

21. Processus de production de fines particules de dioxyde de titane modifiées en surface selon l'une quelconque des revendications 13 à 18, dans lequel la cinquième étape pour la séparation comprend l'étape consistant à disperser les fines particules de dioxyde de titane modifiées en surface dans un solvant aqueux, puis à précipiter les fines particules de dioxyde de titane modifiées en surface à partir d'un solvant organique.
